(19) Europäisches Patentamt
European Patent Office
Office européen des brevets

(11) **EP 1 140 055 B1**

(12) **FASCICULE DE BREVET EUROPEEN**

(45) Date de publication et mention de la délivrance du brevet:
**09.04.2003 Bulletin 2003/15**

(21) Numéro de dépôt: **99958316.4**

(22) Date de dépôt: **14.12.1999**

(51) Int Cl.[7]: **A61K 31/15**, A61K 31/445, A61K 31/454, A61K 31/5375, A61K 31/496, A61P 11/00

(86) Numéro de dépôt international:
**PCT/FR99/03122**

(87) Numéro de publication internationale:
**WO 00/037068 (29.06.2000 Gazette 2000/26)**

(54) **UTILISATION D'UN ANTAGONISTE DES RECEPTEURS 5HT 2A, ET 5HT2A/C POUR LA PREPARATION DE MEDICAMENTS DESTINES AU TRAITEMENT DES RONFLEMENTS ET DE LA SYNDROME DE HAUTE RESISTANCE DES VOIES AERIENNES SUPERIEURES**

VERWENDUNG VON 5HT2A UND 5HT2A/C ANTAGONISTEN ZUR HERSTELLUNG VON ARZNEIMITTELN ZUR BEHANDLUNG DES SCHNARCHENS UND DES HERHOTEN WIDERSTANDES DER OBEREN LUFTWEGE

USE OF A 5HT 2A? AND 5HT 2A/C? RECEPTOR ANTAGONIST FOR PREPARING MEDICINES FOR TREATING SNORING AND HIGH RESISTANCE SYNDROME OF UPPER ANATOMICAL AIRWAYS

(84) Etats contractants désignés:
**AT BE CH CY DE DK ES FI FR GB GR IE IT LI LU MC NL PT SE**
Etats d'extension désignés:
**AL LT LV MK RO SI**

(30) Priorité: **22.12.1998 FR 9816295**

(43) Date de publication de la demande:
**10.10.2001 Bulletin 2001/41**

(73) Titulaire: **SANOFI-SYNTHELABO
75013 Paris (FR)**

(72) Inventeur: **CATTELIN, Françoise
F-75016 Paris (FR)**

(74) Mandataire: **Thouret-Lemaitre, Elisabeth et al
Sanofi-Synthélabo
Service Brevets
174, avenue de France
75013 Paris (FR)**

(56) Documents cités:
**EP-A- 0 373 998**
**WO-A-91/18602**
**WO-A-98/38189**
**WO-A-99/00119**
**WO-A-99/43319**

- **VEASEY S C ET AL: "The effects of serotonin antagonists in an animal model of sleep-disordered breathing" AMERICAN JOURNAL OF RESPIRATORY AND CRITICAL CARE MEDICINE,US,AMERICAN LUNG ASSOCIATION, NEW YORK, NY, vol. 153, no. 2, février 1996 (1996-02), pages 776-786, XP002111045 ISSN: 1073-449X**
- **YOSHIOKA M ET AL: "PHARMACOLOGICAL CHARACTERIZATION OF 5-HYDROXYTRYPTAMINE-INDUCED APNEA IN THE RAT" JOURNAL OF PHARMACOLOGY AND EXPERIMENTAL THERAPEUTICS,US,AMERICAN SOCIETY FOR PHARMACOLOGY AND, vol. 260, no. 2, 1 janvier 1992 (1992-01-01), pages 917-924, XP002063051 ISSN: 0022-3565**
- **RADULOVACKI: "serotonin 5-HT3 receptor antagonist" SLEEP, vol. 21, no. 2, - 1998 pages 131-136, XP002063050**
- **DATABASE BIOSIS [Online] BIOSCIENCES INFORMATION SERVICE, PHILADELPHIA, PA, US REDROBE, JOHN P. ET AL: "Clonidine potentiates the effects of 5-HT1A, 5-HT1B and 5-HT2A/2C antagonists and 8-OH-DPAT in the mouse forced swimming test." retrieved from STN XP002135598 & EUROPEAN NEUROPSYCHOPHARMACOLOGY, (AUG., 1998) VOL. 8, NO. 3, PP. 169-173 ,**

Il est rappelé que: Dans un délai de neuf mois à compter de la date de publication de la mention de la délivrance du brevet européen, toute personne peut faire opposition au brevet européen délivré, auprès de l'Office européen des brevets. L'opposition doit être formée par écrit et motivée. Elle n'est réputée formée qu'après paiement de la taxe d'opposition. (Art. 99(1) Convention sur le brevet européen).



Printed by Jouve, 75001 PARIS (FR)

## Description

La présente invention concerne une nouvelle utilisation des antagonistes de différents récepteurs de la sérotonine, à savoir les antagonistes des récepteurs $5HT_{2A}$ et $5HT_{2A-2C}$ de la sérotonine, de préférence des antagonistes qui sont spécifiques desdits récepteurs.

Parmi ces antagonistes spécifiques des récepteurs $5HT_{2A}$ et $5HT_{2A-2C}$, on distingue plusieurs composés ou familles de composés.

Le 1-(2-fluorophényl)-3-(4-hydroxyphényl)-prop-2-èn-1-one-O-2-diméthylamino éthyl)-oxime de formule (I) et ses sels pharmaceutiquement acceptables, sont décrits dans le brevet européen 0 373 998 B1 comme des antagonistes des récepteurs $5HT_2$ :

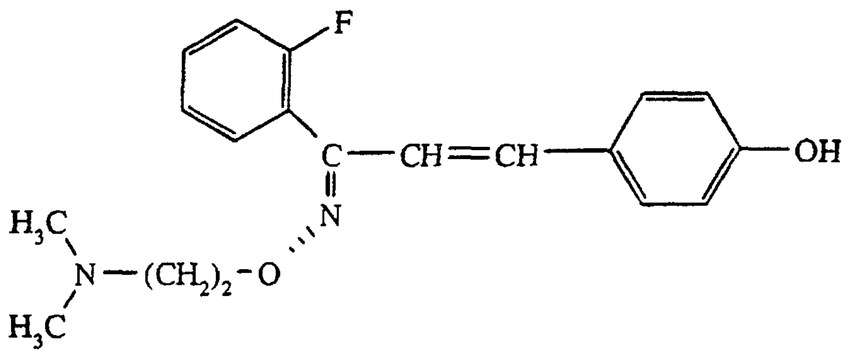

(I)

Plus particulièrement, l'hémifumarate de (1Z,2E)-1-(2-fluorophényl)-3-(4-hydroxyphényl)-prop-2-èn-1-one-O-(2-diméthylaminoéthyl)-oxime, connu sous le nom de code SR 46349B et ci-après dénommé composé A, a été étudié pour ses propriétés biochimiques et pharmacologiques. Le composé A est un antagoniste spécifique du récepteur $5HT_{2A}$, à savoir qu'il n'a pas d'affinité pour les récepteurs $5HT_{1A}$, $5HT_{1B}$ et $5HT_{1D}$ et une affinité modérée pour le récepteur $5HT_{2C}$ ; dans les études sur les tissus isolés, l'absence d'activité du composé A sur le fundus stomachal du rat indique une spécificité $5HT_{2A}$ versus $5HT_{2B}$ (M. Rinaldi-Carmona et al., J. Pharmacol. Exp. Ther., 1992, <u>262,</u> 2, 759-768). Chez le rongeur, on a montré que ce composé se lie de façon majoritaire aux régions du cerveau contenant le récepteur $5HT_2$ (M. Rinaldi-Carmona et al., Life Sciences, 1993, <u>54</u>, 119-127).

Le (+)-(R)-α-(2,3-diméthoxyphényl)-(1-[2-(4-fluoro phényl)éthyl]-4-pipéridin-4-yl) méthanol de formule (II) dont le nom de code est MDL 100907 est connu pour être un antagoniste des récepteurs $5HT_{2A}$ (J. Pharmacol. Exp. Therap., 1996, <u>277,</u> 968-981).

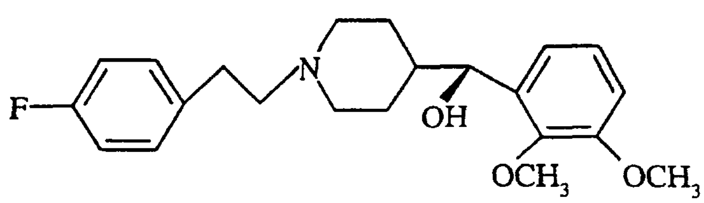

(II)

La demande de brevet internationale WO 98/38189 décrit des dérivés d'oxazolidine de formule (III) ayant des propriétés antagonistes des récepteurs $5HT_{2A}$:

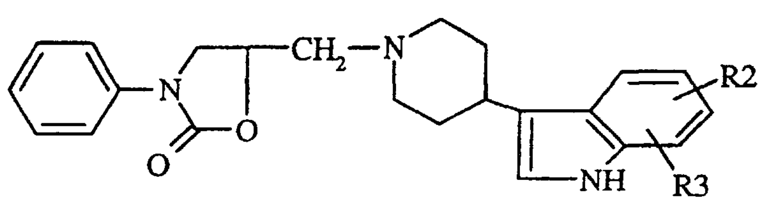

(III)

Le chlorhydrate de (S)-2-[[(7-fluoro-2,3-dihydro-1*H*-inden-4-yl)oxy]méthyl] morpholine dont le nom de code est YM 992 est un antagoniste des récepteurs $5HT_{2A}$ décrit par Takeuchi H. et al. dans Eur. J. Pharmacol. 1997, <u>329,</u> 27-35.

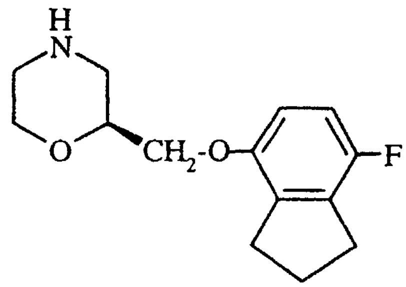

, HCl (IV)

La fananserine de formule :

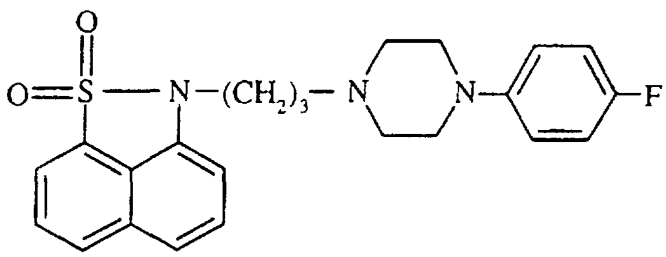

(V)

est également un antagoniste des récepteurs $5HT_{2A}$ décrit par Doble A. et al. dans Br. J. Pharmacol., 1992, 105, 27-36.

Des études sur le sommeil ont montré que certains antagonistes des récepteurs $5HT_2$ comme la ritansérine, l'amoxapine, et ICI 169 369, modifient l'architecture du sommeil et régulent ou augmentent le temps de sommeil lent (G. Loas, L'encéphale, 1991, XVII, 423-425).

Les mécanismes centraux par lesquels la sérotonine module l'activité respiratoire ont été étudiés et l'on a trouvé que parmi les différentes familles de récepteurs, seuls les récepteurs $5HT_1$ et les récepteurs $5HT_2$ affectent le contrôle nerveux des muscles respiratoires (R. Monteau et al., Eur. J. Pharmacol., 1994, 259, 71-74).

Dans le même article, ces auteurs étudient *in-vitro* sur des préparations de tissus de rats nouveau-nés, à l'aide du composé A, quels sous-types de récepteurs interviennent dans la modulation de l'activité respiratoire. Ils observent que le prétraitement par le composé A empêche ou réduit de façon significative l'activité tonique cervicale induite par la 5-hydroxytryptamine et attribuée à l'activation des récepteurs spinaux $5HT_2$ ; de même, il inhibe l'effet dépresseur de la 5-hydroxytryptamine sur l'activité du nerf hypoglosse. Par ailleurs, les auteurs suggèrent que le composé A pourrait être utilisé pour l'étude in-vivo des mécanismes responsables de l'apnée obstructive.

L'utilisation du L-tryptophane, un précurseur de la sérotonine, dans les troubles respiratoires du sommeil a été étudiée chez l'homme (H.S. Schmidt, Bull. Eur. Physiopathol. Respir., 1983, 19, 625-629) ainsi que celle de la fluoxétine, inhibiteur sélectif de la recapture de la sérotonine (Hanzel D.A., Chest, 1991, 100, 416-421).

La demande de brevet européenne EP 449 561 A indique l'utilisation de la (R)-fluoxétine, pour traiter différentes affections dont les apnées du sommeil.

Un article de M. Yoshioka et al. dans J. Pharmacol. Exp. Ther., 1992, 260 (2), 917-924 concerne la caractérisation pharmacologique de l'apnée induite par la 5-HT chez le rat ; il rapporte que des antagonistes du récepteur $5HT_2$ tels que la kétansérine et le méthysergide, inhibent l'apnée et l'augmentation de résistance pulmonaire induites par la 5-HT, et montre qu'un agoniste $5HT_2$ inhibe la respiration de manière identique à la 5-HT. Cet article suggére que l'apnée induite par la 5-HT est en partie médiée par le système vagal.

S.C. Veasey et al. (Am. J. Respir. Crit. Care Med., 1996, 153, 776-786) étudient les effets de deux antagonistes de la sérotonine sur un modèle animal (le bouledogue anglais) des troubles respiratoires du sommeil survenant pendant le sommeil à mouvements oculaires rapides (en anglais : REM : Rapid eye movement). Ils concluent que la ritansérine et le méthysergide qui antagonisent notamment les récepteurs $5HT_2$, administrés de façon systémique, conduisent à une diminution marquée de l'activité du muscle dilatateur des voies respiratoires supérieures et à une faible diminution de l'activité du diaphragme, ces diminutions coïncidant avec les désaturations en oxyhémoglobine. Les auteurs suggèrent que la sérotonine pourrait jouer un rôle dans l'augmentation de l'activité dilatatrice des voies respiratoires supérieures au cours du sommeil à mouvements oculaires rapides. D. Rose et al. (Fundam. Clin. Pharmacol., 1996, 10 (1), 80) rapportent les résultats d'études réalisées in-vivo sur des animaux nouveau-nés décérébrés (rats et chats). Chez le chat, ils observent que l'administration de fortes doses de 5-hydroxytryptamine induit des apnées centrales prolongées liées à des périodes d'expiration active. Chez le rat, ils n'observent aucune apnée après administration de

5-hydroxytryptamine, ce qui est en contradiction avec les résultats observés in-vitro chez le rat nouveau-né.

Les différences interespèces observées, sur les mécanismes respiratoires ainsi que les différences entre les résultats des études in-vivo et in-vitro chez le rat ne donnent aucune indication à l'homme de l'art sur l'effet éventuel des antagonistes spécifiques des récepteurs $5HT_{2A}$ ou $5HT_{2A-2C}$ sur les troubles respiratoires liés au sommeil chez l'homme.

De façon inattendue, on a maintenant trouvé que les antagonistes des récepteurs $5HT_{2A}$ ou $5HT_{2A-2C}$ notamment les composés de formule (I) en particulier le composé A, et le composé de formule (II) sont efficaces dans le traitement des ronflements et du syndrome de haute résistance ou de résistance des voies aériennes supérieures.

Ainsi la présente invention est relative à l'utilisation d'un antagoniste des récepteurs $5HT_{2A}$ ou $5HT_{2A-2C}$, notamment un composé de formule (I) et le composé de formule (II), pour la préparation de médicaments utiles dans le traitement des ronflements et du syndrome de haute résistance ou de résistance des voies aériennes supérieures.

La présente invention est également relative à une composition pharmaceutique pour le traitement des ronflements et du syndrome de haute résistance ou de résistance des voies aériennes supérieures comprenant un antagoniste des récepteurs $5HT_{2A}$ ou $5HT_{2A-2C}$.

De plus, l'invention concerne une méthode de traitement des ronflements et du syndrome de haute résistance ou de résistance des voies aériennes supérieures comprenant l'administration d'une quantité efficace d'un antagoniste des récepteurs $5HT_{2A}$ ou $5HT_{2A-2C}$.

Le syndrome de haute résistance ou de résistance des voies aériennes supérieures (en anglais upper airway resistance syndrome : UARS) a été décrit par C.

Guilleminault et al., dans Chest, 1993, 104 (3), 781-787. Il consiste en des éveils répétés visibles sur l'électroencéphalogramme et s'accompagnant d'une augmentation de l'effort respiratoire, indiquée par une pression oesophagienne négative.

Les conséquences cliniques du syndrome de résistance de voies aériennes supérieures peuvent inclure :

i) une somnolence excessive pendant la journée et secondairement des pertes de productivité, voire des risques d'accidents ;
ii) une fatigue chronique, une irritabilité, une nycturie, des céphalées matinales, des troubles de la mémoire et/ou de la personnalité ;

une augmentation de la susceptibilité aux complications cardiovasculaires telles que l'hypertension pulmonaire, l'insuffisance cardiaque, l'hypertension artérielle systémique, les arythmies cardiaques, les accidents vasculaires cérébraux et l'infarctus du myocarde.

On a maintenant trouvé que les antagonistes des récepteurs $5HT_{2A}$ ou $5HT_{2A-2C}$, préférentiellement les antagonistes spécifiques desdits récepteurs, notamment le composé de formule (I), en particulier le composé A et le composé de formule (II) sont actifs chez l'homme dans le traitement des troubles du sommeil cités ci-dessus.

Chez des sujets sains jeunes (18 à 35 ans), on a observé que l'administration du composé A induit un doublement de la durée des stades 3 et 4 du sommeil lent dès la dose de 1 mg ; les stades 1 et 2 du sommeil lent étant légèrement diminués et le sommeil paradoxal n'étant pas modifié.

L'effet du composé A est déterminé au cours d'une étude clinique réalisée en double aveugle versus placebo à laquelle participent des patients présentant un syndrome de haute résistance des voies aériennes supérieures caractérisé par la présence d'efforts respiratoires avec éveils ou micro-éveils répétés et des symptômes cliniques tels que somnolence diurne et/ou hypertension et/ou fatigue et/ou céphalée matinale et/ou nycturie etc...

Une gélule contenant une dose active du composé A, par exemple 5 mg, est administrée chaque jour avec le repas du soir. On observe une diminution nette des efforts respiratoires et du nombre d'éveils ou micro-éveils ainsi qu'une diminution des symptômes cliniques.

Le composé de formule (I) et ses sels pharmaceutiquement acceptables sont préparés selon la description donnée dans le brevet européen 0 373 998 B1.

Le composé de formule (II) est préparé selon la description donnée dans le brevet européen 0 531 410 B. Les composés de formule (III) est préparé comme décrit dans la demande internationale WO 98/38189.

Le composé de formule (IV) est préparé selon le mode opératoire décrit dans la demande internationale WO 94/18182.

Le composé de formule (V) est préparé selon le procédé décrit dans la demande européenne EP 350 403.

Dans les compositions pharmaceutiques de la présente invention pour l'administration orale, sublinguale, sous-cutanée, intramusculaire, intraveineuse, transdermique, locale ou rectale, le principe actif seul ou en association avec un autre principe actif peut être administré sous forme unitaire d'administration, en mélange avec des supports pharmaceutiques classiques, aux animaux et aux êtres humains. Les formes unitaires d'administration appropriées comprennent les formes par voie orale telles que les comprimés, les gélules, les poudres, les granules et les solutions ou suspensions orales, les formes d'administration sublinguale et buccale, les aérosols, les implants, les formes d'administration sous-cutanée, transdermique, intramusculaire, intraveineuse, intranasale et les formes d'administration rectale.

Le dosage journalier du composé selon l'invention est de 0,001 à 1 mg/kg, avantageusement de 0,002 à 0,5 mg/kg, préférentiellement de 0,005 à 0,2 mg/kg, à administrer en une ou plusieurs fois. Les composés sont généralement formulés en unité de dosage contenant de 0,05 à 50 mg, avantageusement de 0,1 à 25 mg, préférentiellement de 0,2 à 10 mg, de principe actif par unité de dosage, à administrer une fois, deux fois ou plusieurs fois en même temps, selon la nécessité. Bien que ces dosages soient des exemples de situation moyennes, il peut y avoir des cas particuliers où des dosages plus élevés ou plus faibles sont appropriés, de tels dosages appartiennent également à l'invention. Selon la pratique habituelle, le dosage approprié à chaque patient est déterminé par le médecin selon le mode d'administration, l'âge, le poids et la réponse dudit patient.

Lorsque l'on prépare une composition solide sous forme de comprimé, on peut ajouter au principe actif micronisé ou non, un agent mouillant et on mélange le tout avec un véhicule pharmaceutique tel que la silice, l'amidon, le lactose, le stéarate de magnésium, le talc ou analogues. On peut enrober les comprimés de saccharose, de divers polymères ou d'autres matières appropriées ou encore les traiter de telle sorte qu'ils aient une activité prolongée ou retardée et qu'ils libèrent d'une façon continue une quantité prédéterminée de principe actif.

On obtient une préparation en gélules en mélangeant le principe actif ou les principes actifs avec un diluant en incorporant le mélange obtenu dans des gélules molles ou dures.

Une préparation sous forme de sirop ou d'élixir peut contenir le principe actif ou les principes actifs conjointement avec un édulcorant, acalorique de préférence, du méthylparaben et du propylparaben comme antiseptiques, ainsi qu'un agent donnant du goût et un colorant approprié.

Les poudres ou les granules dispersibles dans l'eau peuvent contenir le principe actif en mélange avec des agents de dispersion ou des agents mouillants, ou des agents de mise en suspension, comme la polyvinylpyrrolidone ou polyvidone, de même qu'avec des édulcorants ou des correcteurs du goût.

Pour une administration rectale, on recourt à des suppositoires qui sont préparés avec des liants fondant à la température rectale, par exemple du beurre de cacao ou des polyéthylèneglycols.

Pour une administration parentérale, on utilise des suspensions aqueuses, des solutions salines isotoniques ou des solutions stériles et injectables qui contiennent des agents de dispersion et/ou des agents solubilisants pharmacologiquement compatibles, par exemple le propylèneglycol ou le butylèneglycol.

Ainsi, pour préparer une solution aqueuse injectable par voie intraveineuse on peut utiliser un cosolvant : un alcool tel que l'éthanol, un glycol tel que le polyéthylèneglycol ou le propylèneglycol et un tensioactif hydrophile tel que le polysorbate 80. Pour préparer une solution huileuse injectable par voie intramusculaire, on peut solubiliser le principe actif par un triglycéride ou un ester de glycérol.

Pour l'administration transdermique, on peut utiliser des patches sous forme multilaminée ou à réservoir dans lequel le principe actif est en solution alcoolique.

Le principe actif peut être formulé également sous forme de microcapsules ou microsphères, éventuellement avec un ou plusieurs supports ou additifs.

Le principe actif peut être également présenté sous forme de complexe avec une cyclodextrine, par exemple α -, β- ou γ- cyclodextrine, 2-hydroxypropyl-β-cyclodextrine ou méthyl-β-cyclodextrine.

Parmi les formes à libération prolongée utiles dans le cas de traitements chroniques, on peut utiliser des implants. Ceux-ci peuvent être préparés sous forme de suspension huileuse ou sous forme de suspension de microsphères dans un milieu isotonique.

Selon la présente invention, les formes orales d'administration sont préférées.

EXEMPLE 1 : Gélule à 0,1 mg de (1Z,2E)-1-(2-fluorophényl)-3-(4-hydroxyphényl)-prop-2-èn-1-one-O- (2-diméthylaminoéthyl)-oxime.

| | |
|---|---:|
| Composé A | 0,236 mg |
| Lactose monohydrate extra-fin cristallisé | 99,014 mg |
| Amidon de maïs modifié | 25 mg |
| Silice colloïdale anhydre | 0,11 mg |
| Stéarate de magnésium | 0,64 mg |
| Pour une gélule blanc opaque de taille 0, terminée à | 125 mg |

EXEMPLE 2 : Gélule à 1 mg de (1Z,2E)-1-(2-fluorophényl)-3-(4-hydroxyphényl)-prop-2-èn-1-one-O-(2-diméthylaminoéthyl)-oxime.

| | |
|---|---|
| Composé A | 1,18 mg |
| Lactose monohydrate extra-fin cristallisé | 451,42 mg |
| Amidon de maïs modifié | 114 mg |
| Silice colloïdale anhydre | 0,5 mg |
| Stéarate de magnésium | 2,9 mg |
| Pour une gélule blanc opaque de taille 0, terminée à | 570 mg |

EXEMPLE 3: Gélule à 5 mg de (1Z,2E)-1-(2-fluorophényl)-3-(4-hydroxyphényl)-prop-2-èn-1-one-O-(2-diméthylaminoéthyl)-oxime.

| | |
|---|---|
| Composé A | 5,9 mg |
| Lactose monohydrate extra-fin cristallisé | 446,7 mg |
| Amidon de maïs modifié | 114 mg |
| Silice colloïdale anhydre | 0,5 mg |
| Stéarate de magnésium | 2,9 mg |
| Pour une gélule blanc opaque de taille 0, terminée à | 570 mg |

EXEMPLE 4: Gélule à 10 mg de (1Z,2E)-1-(2-fluorophényl)-3-(4-hydroxyphényl)-prop-2-èn-1-one-O-(2-diméthylaminoéthyl)-oxime.

| | |
|---|---|
| Composé A | 11,8 mg |
| Lactose monohydrate extra-fin cristallisé | 440,8 mg |
| Amidon de maïs modifié | 114 mg |
| Silice colloïdale anhydre | 0,5 mg |
| Stéarate de magnésium | 2,9 mg |
| Pour une gélule blanc opaque de taille 0, terminée à | 570 mg |

EXEMPLE 5: Gélule à 20 mg de formule (+)-(R)-α-(2,3-diméthoxyphényl)-(1-[2-(4-fluorophényl)éthyl]-4-pipéridin-4-yl) méthanol.

| | |
|---|---|
| (+) - (R)-α-(2,3-diméthoxyphényl) - (1-[2- (4-fluorophényl)éthyl]-4-pipéridin-4-yl) méthanol | 20 mg |
| Lactose monohydrate extra-fin cristallisé | 432,6 mg |
| Amidon de maïs modifié | 114 mg |
| Silice colloïdale anhydre | 0,5 mg |
| Stéarate de magnésium | 2,9 mg |
| Pour une gélule blanc opaque de taille 0, terminée à | 570 mg |

## Revendications

1. Utilisation du 1-(2-fluorophényl)-3-(4-hydroxyphényl)-prop-2-èn-1-one-O-2-(diméthylaminoéthyl) -oxime ou d'un de ses sels pharmaceutiquement acceptables pour la préparation de médicaments utiles dans le traitement des ronflements et du syndrome de haute résistance ou de résistance des voies aériennes supérieures.

2. Utilisation selon la revendication 1 de l'hémifumarate de (1Z, 2E)-1-(2-fluorophényl)-3-(4-hydroxyphényl) -prop-2-èn-1-one-O-(2-diméthylaminoéthyl) -oxime.

3. Utilisation selon la revendication 1 ou la revendication 2 pour la préparation de médicaments utiles dans le traitement des ronflements.

4. Utilisation selon la revendication 1 ou la revendication 2 pour la préparation de médicaments utiles dans le traitement du syndrome de haute résistance ou de résistance des voies aériennes supérieures.

## Claims

1. Use of 1-(2-fluorophenyl)-3-(4-hydroxyphenyl)prop-2-en-1-one-O-2-(dimethylaminoethyl)oxime or of one of its pharmaceutically acceptable salts for the proparation of medicines useful in the treatment of snoring and upper airway high-resistance or resistance syndrome.

2. Use according to Claim 1 of (1Z,2E)-1-(2-fluorophenyl)-3-(4-hydroxyphenyl)prop-2-en-1-one-O-(2-dimethylaminoethyl)oxime hemifumarate.

3. Use according to Claim 1 or Claim 2 for the preparation of medicines useful in the treatment of snoring.

4. Use according to Claim 1 or Claim 2 for the preparation of medicines useful in the treatment of upper airway high-resistance or resistance syndrome.

## Patentansprüche

1. Verwendung von 1-(2-Fluorphenyl)-3-(4-hydroxyphenyl)-prop-2-en-1-on-O-2-(dimethylaminoethyl)-oxim oder eines seiner pharmazeutisch annehmbaren Salze für die Herstellung von Arzneimitteln zur Behandlung des Schnarchens und des starken Atemwiderstandsyndroms oder des Widerstands der oberen Luftwege.

2. Verwendung nach Anspruch 1 des Hemifumarats von (1Z. 2E)-1-(2-Fluorphenyl)-3-(4-hydroxyphenyl)-prop-2-en-1-on-O-(2-dimethylaminoethyl)-oxim.

3. Verwendung nach Anspruch 1 oder Anspruch 2 für die Herstellung von Arzneimitteln zur Behandlung des Schnarchens.

4. Verwendung nach Anspruch 1 oder Anspruch 2 für die Herstellung von Arzneimitteln zur Behandlung des starken Atemwiderstandsyndroms oder des Widerstands der oberen Luftwege.